# EUROPEAN PATENT APPLICATION

(11) **EP 1 927 589 A1**
(43) Date of publication of application: **04.06.2008**
(21) Application number: 07254643.5
(22) Date of filing: 30.11.2007
(51) Int. Cl.: C07D 215/56, C07D 401/04, C07D 405/12, C07D 409/12, A61K 31/497, A61K 31/335, A61K 31/353, A61K 31/38, A61P 31/04

(54) **Quinoline derivatives**

(30) Priority: 30.11.2006 IN MU19672006
(71) Applicant: CADILA HEALTHCARE LIMITED, Ahmedebad 380 015, Gujarat (IN)
(72) Inventor: Srivastava, Brijesh K. Cedila Healthcare Ltd., Ahmedabad 380015, Gujarat (IN); Jain, Mukul R. Cadila Healthcare Ltd., Ahmedabad 380015, Gujarat (IN); Patel, Pankaj R. Cadila Healthcare Ltd., Ahmedabad 380015, Gujarat (IN)
(74) Representative: Srinivasan, Ravi Chandran

(57) **Abstract**

The present invention relates to novel compounds of general formula (I), their tautomeric forms, their pharmaceutically acceptable salts and pharmaceutical compositions containing them. The present invention also relates to a process for preparing compounds of general formula (I), their tautomeric forms, their pharmaceutically acceptable salts and pharmaceutical compositions containing them.

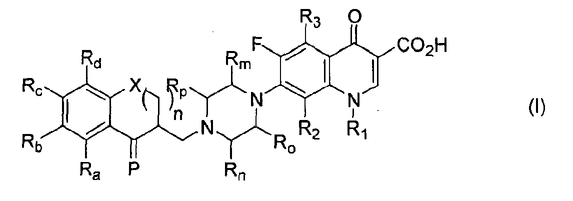

## Description

### Field of Invention

The present invention relates to novel compounds of general formula (I), their tautomeric forms, their pharmaceutically acceptable salts and pharmaceutical compositions containing them. The present invention also relates to a process for preparing compounds of general formula (I), their tautomeric forms, their pharmaceutically acceptable salts and pharmaceutical compositions containing them.

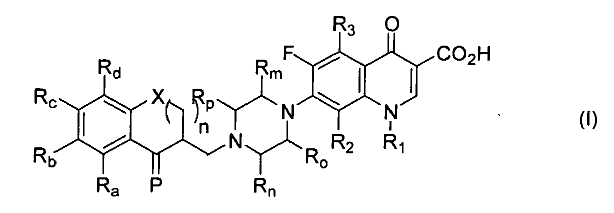

### Background to the invention

Antibiotic resistance is a serious concern worldwide as it would result in strains against which currently available antibacterial agents will be ineffective. In general, bacterial pathogens may be classified as either Gram-positive or Gram-negative pathogens. Antibiotic compounds with effective activity against both Gram-positive and Gram-negative pathogens are generally regarded as having a broad spectrum of activity. The compounds of the present invention though being primarily effective against Gram-positive pathogens are also effective against certain Gram-negative pathogens.

Gram-positive pathogens, for example *Staphylococci, Enterococci, Streptococci* and Mycobacteria, are particularly important because of the development of resistant strains which are both difficult to treat and difficult to eradicate from the hospital environment once established. Examples of such strains are methicillin-resistant *Staphylococcus aureus* (MRSA), methicillin-resistant coagulase negative *staphylococci* (MRCNS), penicillin resistant *Streptococcus pneumoniae* and multiply resistant *Enterococcus faecium,* community acquired pathogens (CAP), and so on.

Quinolones as a class of antibacterial agents are well known and are being used extensively throughout the world. They are potent inhibitors of Gram positive as well as Gram negative pathogens and may be administered orally or intravenously. A wide range of quinolone antibacterials has been introduced in the last decade, which includes norfloxacin, ciprofloxacin, ofloxacin, and the recently launched gatifloxacin and moxifloxacin.

However, some of the quinolone antibacterials have been associated with significant side effects (J. Antimicrob. Chemother., 1994; 33: 685) and some of them have been discontinued at different stages of development (e.g. Trovafloxacin).

The quinolones inhibit bacterial growth by inhibition of DNA gyrase topoisomerase II and topoisomerase IV (Gootz, Medicinal Research, 1996; Rev. 16:433). Quinolone antibacterials and their methods of preparation has been described in WO 0296908, WO 0153273, WO 0132655, WO 0129035, WO 9640190, WO 9640156, WO 9602540, US 4994599, US 4990517, US 4980470, US 4980373, US 4945160, US 4954507, US 4880814, US 4795751, US 4670444, OS 3816119, EP 0805156, EP 0421668, EP 0449445, EP 0300311, EP 0241206, EP 0235762, EP 0167763, EP 0155006, EP 0140116, EP 0028698, DE 3441788, DE 3519286, which are incorporated herein as reference in their entirety. We have also described some quinolone antibacterials in our Indian application no. 462/MUM/2004, which is also incorporated in its entirety as reference.

Fluoroquinoloes having 4,5,6,7-tetrahydrothieno[3,2-c]pyridine moiety at C-7 position with the following structure have been reported [WO0132655] from Abbott laboratory, wherein, the substitution is linked through the carbon atom.

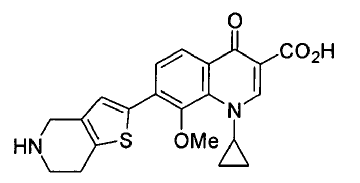

A number of 7-substituted quinolone derivatives have been reported of the following formula, which exhibited significant activities against *Klebsiella pneumoniae,* methicilin resistant *S. aureus,* erythromycin and ampicillin-resistant *Streptococcus pneumoniae* (J. Med. Chem. 2001, 44, 2377-2377).

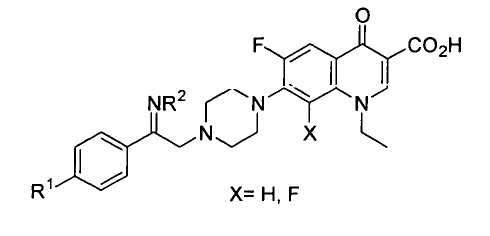

A series of novel 5-amino-6-fluoro-1-[(1*R*,2*S*)-2-fluorocyclopropan-1-yl]-8-methylquinolones bearing fluorinated (3*R*)-3-(1-aminocyclopropan-1-yl)pyrrolidin-1-yl substituents at C-7 position were synthesized by Daiichi Pharmaceuticals ( J. Med. Chem. 2003, 46, 1005-1015) to obtain potent drugs for infections caused by Gram-positive pathogens.

Novel cyano-and amidine-substituted derivatives of thieno [2,3-b] quinolone derivatives have been reported by Ivana Jarak et al. ( Bioorg. Med. Chem. Lett. 2006, 14, 2859-2868).

More recently B. K. Srivastava et al. have reported (Bioorg. Med. Chem. Lett. 2007, 17, 1924-1929)4,5,6,7-tetrahydro-thieno[3,2-c]pyridine quinolones of following formula.

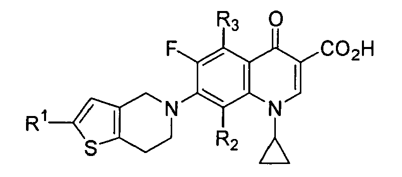

The compounds disclosed in this paper showed *in vitro* antibacterial activities against both the susceptible and resistant strains of bacteria. Few compounds were found to be active against the resistant strains such as methicilin resistant *staphylococcus aureus,* vancomycin resistant *enterococcus faecalis,* and penicillin resistant *streptococcus pneumoniae* as well as against linezolid resistant *staphylococcus aureus.*

However, due to increase in antibacterial resistance and also otherwise there is a continuing need for discovering compounds which are more effective against resistant bacteria, have improved intestinal absorption, metabolic stability, and exhibit less toxicity.

### Summary of the invention

The present invention describes a group of novel compounds useful as antibacterial agents. The novel compounds are defined by the general formula (I) below:

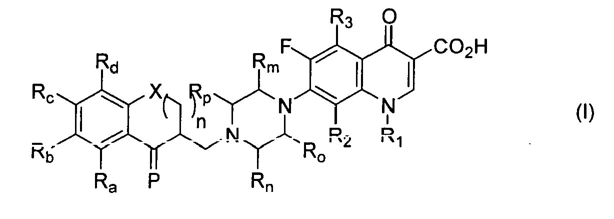

The compounds of the present invention are useful in the treatment of mammals, as preventives and therapeutic agents for infectious diseases. The compounds of this invention have excellent antimicrobial action against various human and veterinary pathogens including but not limited to multiply-resistant staphylococci and streptococci, as well as anaerobic organisms including those of the bacteroides and clostridia species, and acid-fast *Mycobacterium tuberculosis* and *Mycobacterium avium* with better efficacy, potency and minimum toxic effects. The compounds of the present invention have shown more bioavailability than the marketed fluoroquinolones such as Ciprofloxacin, Norfloxacin and Gatifloxacin etc.

### Embodiments of the Invention:

Among many embodiments of the present invention are included novel compounds of general formula (I), their tautomeric forms, novel intermediates involved in their synthesis, their pharmaceutically acceptable salts, their pharmaceutically acceptable solvates and pharmaceutical compositions containing them or their mixtures suitable in the treatment of infectious diseases and processes for their preparation.

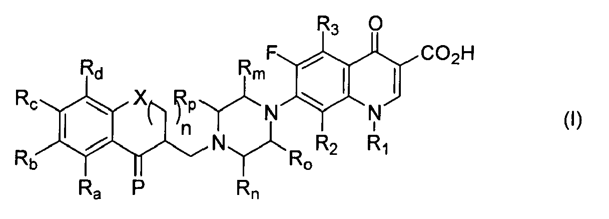

### Detailed Description of the invention:

The novel compounds of the present invention are defined by the general formula (I) as shown below: 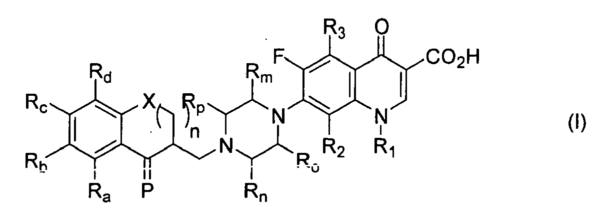 wherein R₁ represents hydrogen, linear or branched, substituted or unsubstituted groups selected from (C₁-C₁₂) alkyl, (C₃-C₁₂)cycloalkyl groups; R₂ & R₃ may be same or different and independently represent H, OH, halogen, or groups selected from NO₂, CN;
Rₘ, Rₙ, Rₒ and Rₚ may be same or different and independently represents hydrogen or optionally substituted alkyl groups;
Rₐ, R_{b}, R_{c}, R_{d} may be same or different and independently represents hydrogen, halogen, haloalkyl, hydroxy, thio, , nitro, cyano, or substituted or unsubstituted groups selected from amino, linear or branched (C₁-C₁₂)alkyl, (C₁-C₁₂)alkoxy derivatives of sulfenyl and sulfonyl groups, sulfonic acid and its derivatives;
'P' represents oxygen, sulfur or the group representing -NR, where R represents an -H or a (C₁-C₃) alkyl group which may be optionally substituted;
'X' may be absent or present and if present, represents the groups -CH₂-, -O-, -S, SO or SO₂; n represents an integer from 0-3.

The term "substituted" used in combination with other radicals, denotes suitable substituents on that radical such as substituted alkyl, substituted cycloalkyl, substituted aryl, etc, mentioned anywhere in the specification. The suitable substituents include, but are not limited to the following radicals, alone or in combination with other radicals, such as, hydroxyl, halo, thio, nitro, amino, cyano, alkyl, haloalkyl, alkoxy, haloalkoxy, cycloalkyl, aryl, hydroxyl amino, sulfenyl derivatives, sulfonyl derivatives, sulfonic acid and its derivatives, phosphonic acid and its derivatives.

When any of the substituents are further substituted, the substituents may be selected from the following radicals, alone or in combination with other radicals, such as, hydroxyl, halo, thio, nitro, amino, acyl, cyano, alkyl, haloalkyl, alkoxy, cycloalkyl, aryl, aryloxy, sulfenyl derivatives, sulfonyl derivatives, sulfonic acid and its derivatives, phosphonic acid and its derivatives

The term "alkyl" used herein, either alone or in combination with other radicals, denotes a linear or branched radical containing one to twelve carbons, such as methyl, ethyl, *n*-propyl, iso-propyl, *n*-butyl, *sec*-butyl, *tert*-butyl, amyl, *t*-amyl, *n*-pentyl , *n*-hexyl, iso-hexyl, heptyl, octyl and the like.

The term "alkenyl" used herein, either alone or in combination with other radicals, denotes a linear or branched radical containing two to twelve carbons; such as vinyl, allyl, 2-butenyl, 3-butenyl, 2-pentenyl, 3-pentenyl, 4-pentenyl, 2-hexenyl, 3-hexenyl, 4-hexenyl, 5-hexenyl, 2-heptenyl, 3-heptenyl, 4-heptenyl, 5-heptenyl, 6-heptenyl and the like. The term "alkenyl" includes dienes and trienes of straight and branched chains.

The term "alkynyl" used herein, either alone or in combination with other radicals, denotes a linear or branched radical containing two to twelve carbons, such as ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 3-hexynyl, 4-hexynyl, 5-hexynyl, and the like. The term "alkynyl" includes di- and tri-ynes.

The term "cycloalkyl" used herein, either alone or in combination with other radicals, denotes a radical containing three to seven carbons, such as cyclopropyl, cyclobutyl, cyclopentyl, cyclohexyl, cycloheptyl and the like.

The term "alkoxy" used herein, either alone or in combination with other radicals, denotes a radical alkyl, as defined above, attached directly to an oxygen atom, such as methoxy, ethoxy, *n*-propoxy, *iso*-propoxy, *n*-butoxy, t-butoxy, *iso*-butoxy, pentyloxy, hexyloxy, and the like.

The term "halo" or "halogen" used herein, either alone or in combination with other radicals, such as "haloalkyl", "perhaloalkyl" etc refers to a fluoro, chloro, bromo or iodo group. The term "haloalkyl" denotes an alkyl radical, as defined above, substituted with one or more halogens; such as fluoromethyl, difluoromethyl, trifluoromethyl, fluoroethyl, difluoroethyl, trifluoroethyl, mono or polyhalo substituted methyl, ethyl, propyl, butyl, pentyl or hexyl groups.

The term "haloalkoxy" denotes a haloalkyl, as defined above, directly attached to an oxygen atom, such as fluoromethoxy, chloromethoxy, fluoroethoxy chloroethoxy groups, and the like.

The term "aryl" or "aromatic" used herein, either alone or in combination with other radicals, denotes an aromatic system containing one, two or three rings wherein such rings may be attached together in a pendant manner or may be fused, such as phenyl, naphthyl, tetrahydronaphthyl, indane, biphenyl, and the like. The term 'aralkyl" denotes an alkyl group, as defined above, attached to an aryl, such as benzyl, phenethyl, naphthylmethyl, and the like. The term "aryloxy" denotes an aryl radical, as defined above, attached to an alkoxy group, as defined above, such as phenoxy, naphthyloxy and the like, which may be substituted. The term "aralkoxy" denotes an arylalkyl moiety, as defined above, attached directly to an oxygen atom, such as benzyloxy, phenethyloxy, naphthylmethyloxy, phenylpropyloxy, and the like, which may be substituted.

The term "heterocyclyl" or "heterocyclic" used herein, either alone or in combination with other radicals, denotes saturated, partially saturated and unsaturated ring-shaped radicals, the heteroatoms selected from nitrogen, sulfur and oxygen. Examples of saturated heterocyclic radicals include but not limited to aziridinyl, azetidinyl, pyrrolidinyl, imidazolidinyl, piperidinyl, piperazinyl, 2-oxopiperidinyl, 4-oxopiperidinyl, 2-oxopiperazinyl, 3-oxopiperazinyl, morpholinyl, thiomorpholinyl, 2-oxomorpholinyl, azepinyl, diazepinyl, oxapinyl, thiazepinyl, oxazolidinyl, thiazolidinyl, and the like; examples of partially saturated heterocyclic radicals include dihydrothiophene, dihydropyran, dihydrofuran, dihydrothiazole, and the like.

The term "heteroaryl" or "heteroaromatic" used herein, either alone or in combination with other radicals, denotes unsaturated 5 to 6 membered heterocyclic radicals containing one or more hetero atoms selected from O, N or S, attached to an aryl group, such as pyridyl, thienyl, furyl, pyrrolyl, oxazolyl, thiazolyl, imidazolyl, isoxazolyl, oxadiazolyl, tetrazolyl, benzopyranyl, benzofuranyl, benzothienyl, indolinyl, indolyl, quinolinyl, pyrimidinyl, pyrazolyl, quinazolinyl, pyrimidonyl, benzoxazinyl, benzoxazinonyl, benzothiazinyl, benzothiazinonyl, benzoxazolyl, benzothizaolyl, benzimidazolyl, and the like.

The term "acyl" used herein, either alone or in combination with other radicals, denotes a radical containing one to eight carbons such as formyl, acetyl, propanoyl, butanoyl, iso-butanoyl, pentanoyl, hexanoyl, heptanoyl, benzoyl and the like, which may be substituted.

The term "acyloxy" used herein, either alone or in combination with other radicals, denotes a radical acyl, as defined above, directly attached to an oxygen atom, such as acetyloxy, propionyloxy, butanoyloxy, iso-butanoyloxy, benzoyloxy and the like.

The term "mono-substituted amino" used herein, either alone or in combination with other radicals, denotes an amino group, substituted with one group selected from (C₁-C₆)alkyl, substituted alkyl, aryl, substituted aryl or arylalkyl groups. Examples of monoalkylamino group include methylamine, ethylamine, *n*-propylamine, *n*-butylamine, *n*-pentylamine and the like.

The term 'disubstituted amino" used herein, either alone or in combination with other radicals, denotes an amino group, substituted with two radicals that may be same or different selected from (C₁-C₆)alkyl, substituted alkyl, aryl, substituted aryl, or arylalkyl groups, such as dimethylamino, methylethylamino, diethylamino, phenylmethyl amino and the like.

The term "alkylthio" used herein, either alone or in combination with other radicals, denotes a straight or branched or cyclic monovalent substituent comprising an alkyl group of one to twelve carbon atoms, as defined above, linked through a divalent sulfur atom having a free valence bond from the sulfur atom, such as methylthio, ethylthio, propylthio, butylthio, pentylthio and the like. Examples of cyclic alkylthio are cyclopropylthio, cyclobutylthio, cyclopentylthio, cyclohexylthio and the like, which may be substituted.

The term "thioalkyl" used herein, either alone or in combination with other radicals, denotes an alkyl group, as defined above, attached to a group of formula -SR', where R' represents hydrogen, alkyl or aryl group, e.g. thiomethyl, methylthiomethyl, phenylthiomethyl and the like, which may be substituted.

The term "sulfenyl" or "sulfenyl and its derivatives" used herein, alone or in combination with other radicals, denotes a bivalent group, -SO- or RSO, where R is substituted or unsubstituted alkyl, aryl, heteroaryl, heterocyclyl, and the like.

The term "sulfonyl" or "sulfones and its derivatives" used herein, either alone or in combination with other radicals, with other terms such as alkylsulfonyl, denotes divalent radical -SO₂-, or RSO₂-, where R is substituted or unsubstituted groups selected from alkyl, aryl, heteroaryl, heterocyclyl, and the like. "Alkylsulfonyl" denotes alkyl radicals, as defined above, attached to a sulfonyl radical, such as methylsulfonyl, ethylsulfonyl, propylsulfonyl and the like. The term "sulfonic acid or its derivatives", used herein, either alone or in combination with other radicals, denotes -SO₃H group and its derivatives such as sulfonylamino(SO₂NH₂); N-alkylaminosulfonyl and N,N-dialkylaminosulfonyl radicals where the sulfonylamino group is substituted with one and two alkyl groups respectively, such as N-methylaminosulfonyl, N-ethylaminosulfonyl, N,N-dimethylaminosulfonyl, N-methyl-N-ethylaminosulfonyl and the like; N-arylaminosulfonyl and N-alkyl-N-arylaminosulfonyl groups where the sulfonylamino group is substituted with one aryl radical, or one alkyl and one aryl radical; -SO₃R, wherein 'R' represents alkyl, aryl, aralkyl groups, as defined above, which may be substituted.

The term "phosphonic acid or its derivatives", used herein, either alone or in combination with other radicals, denotes P(O)(OH)₂, P(O)(O(C₁-C₆) alkyl)₂, P(O)(O aryl)₂, P(O)(OH)(O(C₁-C₆)alkyl), and the like.

Suitable groups and substituents on the groups may be selected from those described anywhere in the specification.
Particularly useful compounds may be selected from but not limited to 1-Cyclopropyl-6-fluoro-7-[4-(6-methoxy-1-oxo-1,2,3,4-tetrahydro-naphthalen-2-yl methyl)-piperazin-1-yl]-4-oxo-1,4-dihydro-quinoline-3-carboxylic acid;
1-Cyclopropyl-6-fluoro-8-methoxy-7-[4-(6-methoxy-1-oxo-1,2,3,4-tetrahydro-naphthalen-2-ylmethyl)-piperazin-1-yl]-4-oxo-1,4-dihydro-quinoline-3-carboxylic acid;
1-Cyclopropyl-5,6,8-trifluoro-7-[4-(6-methoxy-1-oxo-1,2,3,4-tetrahydro-naphthalen-2-ylmethyl)-piperazin-1-yl]-4-oxo-1,4-dihydro-quinoline-3-carboxylic acid;
1-Cyclopropyl-6-fluoro-8-methoxy-7-[4-(6-methoxy-1-oxo-1,2,3,4-tetrahydro-naphthalen-2-ylmethyl)-3-methyl-piperazin-1-yl]-4-oxo-1,4-dihydro-quinoline-3-carboxylic acid;
1-Ethyl-6-fluoro-7-[4-(6-methoxy-1-oxo-1,2,3,4-tetrahydro-naphthalen-2-ylmethyl)-piperazin-1-yl]-4-oxo-1,4-dihydro-quinoline-3-carboxylic acid;
7-[4-(8-Chloro-5-oxo-2,3,4,5-tetrahydro-benzo[b]oxepin-4rylmethyl)-piperazin-l-yl]-l-cyclopropyl-6-fluoro-4-oxo-1,4-dihydro-quinoline-3-carboxylic acid;
7-[4-(8 -Chloro- 5 -oxo-2,3,4,5 -tetrahydro -benzo [b] oxepin-4-yl methyl)-piperazin-1-yl] -1-ethyl -6-fluoro-4-oxo-1,4-dihydro-quinoline-3-carboxylic acid;
7-[4-(7-Chloro-4-oxo-chroman-3-ylmethyl)-piperazin-1-yl]-1-cyclopropyl-6-fluoro-4-oxo-1,4-dihydro-quinoline-3-carboxylic acid;
7-[4-(7-Chloro-4-oxo-chroman-3-ylmethyl)-piperazin- 1 -yl]- 1-ethyl-6-fluoro-4-oxo- ,4-dihydroquinoline-3-carboxylic acid;
7-[4-(5-Chloro-1-oxo-indan-2-ylmethyl)-piperazin-1-yl]-1-cyclopropyl-6-fluoro-4-oxo-1,4-dihydro-quinoline-3-carboxylic acid;
7-[4-(8-Chloro-5-oxo-2,3,4,5-tetrahydro-benzo[b]thiepin-4-ylmethyl)-piperazin-1-yl]-1-cyclopropyl-6-fluoro-4-oxo-1,4-dihydro-quinoline-3-carboxylic acid;
7-[4-(8-Chloro-5-oxo-2,3,4,5-tetrahydro-benzo[b]thiepin-4-ylmethyl)-piperazin-1-yl]-1-ethyl-6-fluoro-4-oxo-1,4-dihydro-quinoline-3-carboxylic acid;
1-Cyclopropyl-6-fluoro-7-[4-(-hydroxyimino-6-methoxy-1,2,3,4-tetrahydro-naphthalen-2-ylmethyl)-piperazin-1-yl]-4-oxo-1,4-dihydro-quinoline-3-carboxylic acid;
Several synthetic routes may be employed to prepare the compounds of the present invention.

The compounds of formula (Ia) can be synthesized using the methods described in Indian Journal of Chemistry, 1996, 35B, 495-498 below, together with conventional techniques known to those skilled in the art, or variations thereon as appreciated by those skilled in the art. Referred methods include, but are not limited to those described below.

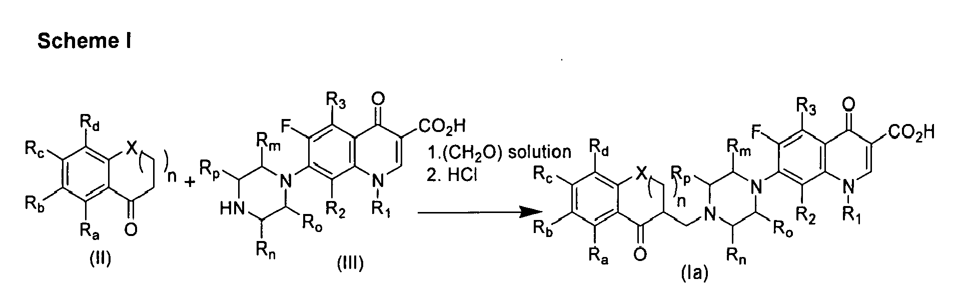

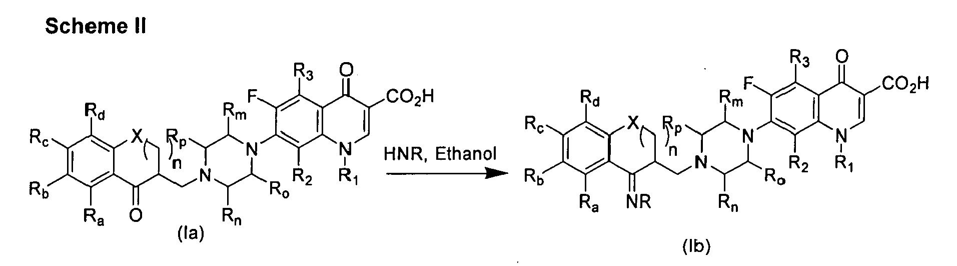

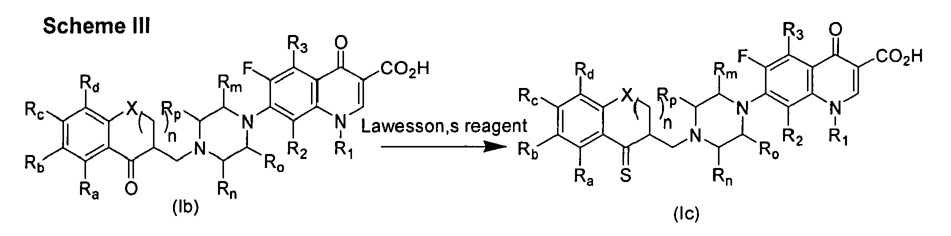

### Scheme I:

Step 1: A compound of general formula (III) may be stirred with equimolar amounts of 35-40 % aqueous formaldehyde solution in suitable solvent(s) at suitable temperature. The resulting mixture is concentrated after completion of the reaction.
Step 2: The resulting residue from step 1 is taken in suitable solvent(s) and treated with HCl. The solvent(s) are removed under reduced pressure and the residue is treated with a suitable ketone of general formula (II) in suitable solvent(s), and the resulting mixture is refluxed. The compounds of formula (Ia) are isolated. Suitable solvent(s) may be selected from alcohols such as methanol, ethanol, propanol, isopropanol, butanol, isobutanol, t-butanol and the like or mixtures thereof.

### Scheme II:

A compound of general formula (Ia) is stirred with equimolar amounts of suitable amine in suitable solvent(s) such as methanol, ethanol, propanol, isopropanol, butanol, isobutanol, t-butanol and the like or mixtures thereof at suitable temperature. The resulting mixture is concentrated after completion of the reaction to afford further compounds of general formula (1b).

### Scheme III:

A compound of general formula (Ia) is refluxed with equimolar amount of Lawesson's reagent in suitable solvent(s) such as THF, toluene, propanol, isopropanol the like or mixtures thereof at suitable temperature. The resulting mixture is concentrated after completion of the reaction. The purification of the crude compound affords compounds of general formula (1c).

Alternatively the compounds of present invention can also be synthesized by method exemplified below:

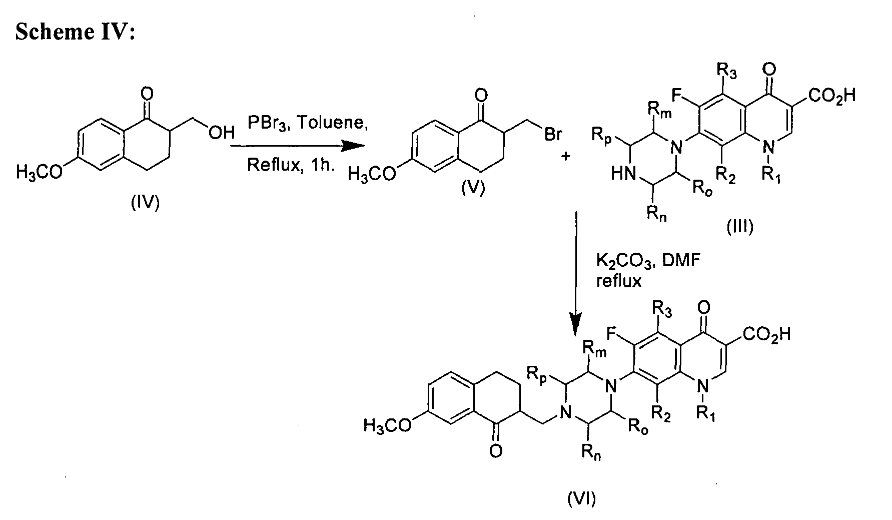

### Scheme IV:

To a solution of 2-Hydroxymethyl-6-methoxy-3,4-dihydro-2H-naphthalen-1-one (IV), which can be synthesized as per the procedure known in the literature (Tetrahedron Letters, 13, 2375-2378, 1992) (0.1 g, 7.3 mole) in toluene (10 mL) was added drop wise phosphorus tribromide (0.59 mL, 6.30 mole) at 25-27 °C. The reaction mixture was refluxed for 1 h. The reaction was monitored by TLC. The solvents were removed under reduced pressure to afford 2-bromomethyl-6-methoxy-3,4-dihydro-2H-naphthalen-1-one (V) (1.3 g, 100%). Compound (V) was further reacted with compound of general formula (III) in presence of base such as K₂CO₃, Na₂CO₃, Cs₂CO₃ etc in solvents such as acetone, DMF, THF etc under reflux condition to afford compound of general formula (VI).

It will be appreciated that in any of the above mentioned reactions any reactive group in the substrate molecule may be protected, according to conventional chemical practice. Suitable protecting groups in any of the above mentioned reactions are those used conventionally in the art. The methods of formation and removal of such protecting groups are those conventional methods appropriate to the molecule being protected. T. W. Greene and P. G. M. Wuts "Protective groups in Organic Synthesis", John Wiley & Sons, Inc, 1999, 3rd Ed., 201-245 along with references therein gives such conventional methods and are incorporated herein as references.

### Determination of Antibacterial activity:

The minimum inhibitory concentrations (MIC's) of the compounds for the microorganisms listed in Table A were determined by preparing working solution for each compound of concentration of 128 µg/ml after dissolving it in DMSO. Two-fold serial dilution of the above solution was prepared in duplicates, using Mueller Hinton Broth, in 96 well Tissue culture plate with cover flat bottom wells to give a final volume of 150 µg/ml and concentration of compound ranging from 64 µg/ml-0.12 µg/ml. 30 µg/ml of Standard suspension of each organism which was prepared with turbidity equivalent to the 1:10 diluted 0.5 McFarland standard with density 10⁷ CFU/ml, was added to each well to get approximately a density of 10⁵ CFU/ml. These 96-well Tissue culture plate containing the test samples and positive and negative controls, were incubated at 37°C for 16-18h.The wells were visually inspected for growth and were also read at 630 nm by Automated Microplate Reader [(EL800) Trinity biotech.] and the MIC's were recorded as the lowest concentration of drug which inhibits the growth of bacteria. The compounds inhibited the growth of these bacteria with MIC's in a range of about 0.125 µg/ml to about 8 µg/ml.

Thus the compounds are useful for treating bacterial infections such as, but not limited to, those shown below in Table A.

**Table A**

| Microorganism |
|---|
| *Bacillus aureus* (MTCC-430) |
| *Staphylococcus aureus* (ATCC-25923) |
| *Staphylococcus aureus* (ATCC-29213) |
| *Enterococcus faecalis* (ATCC-14506) |
| *Klebsiella pneumoniae* (ATCC-10031) |
| *Pseudomonas aeruginosa* (ATCC-27853) |

The compounds of the present invention may optionally be converted to their pharmaceutically acceptable salts.

The novel compounds of the present invention or their pharmaceutically acceptable salts can be formulated into suitable pharmaceutically acceptable compositions by combining with suitable excipients as are well known.

The compounds of Formula (I) are useful in the treatment of microbial infections in mammals, by oral, topical or parenteral administration.

For the treatment of any of the above-mentioned diseases the compounds of formula (I) may be administered, for example, orally, topically, parenterally, in dosage unit formulations containing conventional non-toxic pharmaceutically acceptable carriers, adjuvants and vehicles.

The pharmaceutical composition is provided by employing conventional techniques. Preferably the composition is in unit dosage form containing an effective amount of the active component, that is, the compounds of formula (I) according to this invention.

The quantity of active component, that is, the compounds of formula (I) according to this invention, in the pharmaceutical composition and unit dosage form thereof may be varied or adjusted widely depending upon the particular application method, the potency of the particular compound and the desired concentration. Generally, the quantity of active component will range between 0.5 % -90% by weight of the composition.

The present invention is described in detail by the following examples which are provided for the sake of illustrations only and should not be in any way construed to limit the scope of the invention.
*1H NMR spectral data given in the tables (vide infra) are recorded using a 300 MHz spectrometer (Bruker A VANCE-300) and reported in δ scale. Until and otherwise mentioned the solvent used for NMR is CDCl₃ using Tetramethyl silane as the internal standard*

### Example 1

### 1-Cyclopropyl-6-fluoro-7-[4-(6-methoxy-1-oxo-1,2,3,4-tetrahydro-naphthalen-2-yl methyl)-piperazin-1-yl]-4-oxo-1,4-dihydro-quinoline-3-carboxylic acid (Compound 1)

A cold solution of 1-cyclopropyl-6-fluoro-4-oxo-7-piperazin-1-yl-1,4-dihydro-quinoline-3-carboxylic acid (2 g) in methanol (3 mL) was added gradually under stirring to a cold solution of 35 % formaldehyde solution (3 mL) in methanol. The reaction mixture was stirred at 5 °C for about 30 min. and then kept at 0-5 °C for about 12 h. The solvents were removed under reduced pressure and the residue obtained was taken in methanol and cooled to 0-5 °C. To this cooled mixture was added ethereal HCl (10 mL) and stirred for 15 min. The solvents were removed on a rotatory evaporator and a solution of 6-methoxy-α-tetralone (0.975 g) in methanol was added to it .The reaction mixture was refluxed at 60-65 °C for about 24 h and the reaction was monitored by TLC. The reaction mixture was cooled to 27-28 °C and poured in water, the precipitated solid was filtered on a Buchner funnel and washed with methanol and dried well to obtain the desired compound as a yellow solid (2.3 g, 75 %).
¹H NMR (300 MHz, CDCl₃): δ 15.05 (br s, 1H), 8.70 (s, 1H), 8.03 (d, *J* = 4.29 Hz, 1H), 7.99 (s, 1H), 7.3 (d, *J* = 7.14 Hz, 1H), 6.83 (dd, *J =* 8.73 and 2.43 Hz, 1H ), 6.70 (d, *J* = 2.22 Hz, 1H), 3.87 (s, 3H), 3.53 (m, 1H), 3.36 (br s, 4H), 3.03 (m, 1H), 3.01 (m, 2H), 2.77 (m, 2H), 2.64 (br s, 4H), 2.36 (m, 1H), 1.61(m, 1H), 1.35 (m, 2H), 1.20 (m, 2H).

The following compounds were prepared as per the processes similar to those disclosed in the above example. Suitable modifications, alterations which may be required are considered to be well within the scope of a person skilled in the art.

### Example 2

### 1-Cyclopropyl-6-fluoro-8-methoxy-7-[4-(6-methoxy-1-oxo-1,2,3,4-tetrahydro-naphthalen-2-ylmethyl)-piperazin-1-yl]-4-oxo-1,4-dihydro-quinoline-3-carboxylic acid (Compound 2)

¹H NMR (300 MHz, DMSO-*d*₆): δ 14.84 (br s, 1H), 8.72 (s, 1H), 7.89-7.87 (m, 2H), 6.93 (m, 2H), 4.17 (m, 1H), 3.84-3.80 (m, 7H), 3.68-3.59 (m, 7H), 3.55 (m, 1H), 3.06 (m, 1H), 3.03 (m, 2H), 2.79 (m, 2H), 1.63(m, 1H), 1.37 (m, 2H), 1.24 (m, 2H).

### Example 3

### 1-Cyclopropyl-5,6,8-trifluoro-7-[4-(6-methoxy-1-oxo-1,2,3,4-tetrahydro-naphthalen-2-ylmethyl)-piperazin-1-yl]-4-oxo-1,4-dihydro-quinoline-3-carboxylic acid (Compound 3)

¹H NMR (300 MHz, DMSO-*d*₆): δ 14.71 (br s, 1H), 8.63 (s, 1H), 7.81 (d, *J* = 8.76 Hz, 1H), 6.87.(m, 2H), 4.09 (m, 1H), 3.81 (s, 3H), 3.39-3.19 (br s, 4H), 2.95 (m, 2H), 2.62-2.55 (m, 2H), 2.49 (br s, 4H), 2.31-2.26 (m, 2H), 1.80 (m, 1H), 1.13 (d, *J* = 5.46 Hz, 4H).

### Example 4

### 1-Cyclopropyl-6-fluoro-8-methoxy-7-[4-(6-methoxy-1-oxo- 1,2,3,4-tetrahydro-naphthalen-2-ylmethyl)-3-methyl-piperazin-1-yl]-4-oxo-1,4-dihydro-quinoline-3-carboxylic acid (Compound 4)

¹H NMR (CDCl₃+ few drops of CD₃OD): δ 8.48 (s, 1H), 7.95 (dd, *J* = 10.26, 8.80 Hz, 2H), 6.84 (d, J=8.82. Hz, 1H), 6.74 (s, 1H), 4.19-4.10 (m, 2H), 4.05-3.90 (m, 2H), 3.82 (s, 3H), 3.79 (s, 3H), 3.71 (s, 2H), 3.62-3.55(m, 2H), 3.49(d, *J* = 4.77 Hz, 2H), 3.04 (s, 2H), 2.80-2.50 (m, 1H), 2.07-2.03 (s, 1H), 1.65 (d, J=6.15 Hz, 3H), 1.25-1.23 (m, 3H), 1.05-1.02 (m, 2H), 0.95 (m, 1H).

### Example 5

### 1-Ethyl-6-fluoro-7-[4-(6-methoxy-1-oxo-1,2,3,4-tetrahydro-naphthalen-2-ylmethyl)-piperazin-1-yl]-4-oxo-1,4-dihydro-quinoline-3-carboxylic acid (Compound 5)

¹H NMR (300 MHz, CDCl₃): δ 15.14 (br s, 1H), 8.65 (s, 1H), 8.00 (m, 2H), 6.82 (dd, *J* = 8.88 and 2.31 Hz, 2H), 6.70 (d, *J* = 2.96 Hz, 1H), 4.31 (q, 2H), 3.86 (s, 3H), 3.55 (br s, 4H), 3.00 (m, 3H), 2.81 (m, 2H), 2.77 (br s, 4H), 2.62 (m, 1H), 2.36 (m, 1H), 1.58 (t, 3H).

### Example 6

### 7-[4-(8-Chloro-5-oxo-2,3,4,5-tetrahydro-benzo[b]oxepin-4-ylmethyl)-piperazin-1-yl]-1-cyclopropyl-6-fluoro-4-oxo-1,4-dihydro-quinoline-3-carboxylic acid (Compound 6)

¹H NMR (300 MHz, DMSO-*d*₆): δ 10.86 (br s, 1H), 8.67 (s, 1H), 7.94 (d, *J* = 15 Hz, 1H), 7.68 (d, *J* = 6 Hz, 1H), 7.57 (d, *J* = 6 Hz, 1H), 7.26-7.22 (m, 2H), 4.46 (m, 1H), 4.02 (m, 1H), 3.14 (br s, 4H), 3.01 (m, 3H), 2.77 (m, 1H), 2.55 (br s, 4H), 1.75 (m, 1H) 1.61(m, 1H), 1.35 (m, 2H), 1.20 (m, 2H).

### Example 7

### 7-[4-(8-Chloro-5-oxo-2,3,4,5-tetrahydro-benzo[b]oxepin-4-ylmethyl)-piperazin-1-yl]-1-ethyl-6-fluoro-4-oxo-1,4-dihydro-quinoline-3-carboxylic acid (Compound 7)

¹H NMR (300 MHz, DMSO-d₆): δ 15.33 (br s, 1H), 8.93 (s, 1H), 7.88 (d, *J* = 13.32 Hz, 1H), 7.74 (d, *J* = 8.28 Hz, 1H), 7.16 (m, 3H ), 4.56 (m, 2H), 4.46 (m, 1H), 4.02 (m, 1H), 3.14 (br s, 4H), 3.01 (m, 3H), 2.77 (m, 1H), 2.55 (br s, 4H), 1.75 (m, 1H), 1.37 (t, 3H).

### Example 8

### 7-[4-(7-Chloro-4-oxo-chroman-3-ylmethyl)-piperazin-yl]-1-cyclopropyl-6-fluoro-4-oxo-1,4-dihydro-quinoline-3-carboxylic acid (Compound 8)

¹H NMR (300 MHz, DMSO-*d*₆): δ 14.80 (br s, 1H), 8.63 (s, 1H), 7.98 (d, *J* = 15 Hz, 1H), 7.65 (d, *J* = 6 Hz, 1H), 7.51 (d, *J* = 6 Hz, 1H ), 7.29-7.25 (m, 1H), 4.40 (m, 1H), 4.05 (m, 1H), 3.61 (s, 3H), 3.18 (br s, 4H), 3.03 (m, 3H), 2.52 (br s, 4H), 1.60(m, 1H), 1.36 (m, 2H), 1.21 (m, 2H).

### Example 9

### 7-[4-(7-Chloro-4-oxo-chroman-3-ylmethyl)-3-methyl-piperazin-1-yl]-1-cyclopropyl-6-fluoro-8-methoxy-4-oxo-1,4-dihydro-quinoline-3-carboxylic acid (Compound 9)

¹H NMR (300 MHz, DMSO-*d*₆): δ 14.08 (br s, 1H), 8.55 (s, 1H), 7.98 (dd, *J =* 10.26, 8.80 Hz, 2H), 6.97 (d, J=8.82 Hz, 1H), 6.814 (s, 1H), 4.41 (m, 1H), 4.06 (m, 1H), 3.79 (s, 3H), 3.56 (m, 1H), 3.11 (br s, 4H), 3.00 (m, 2H), 2.51 (br s, 4H), 1.58 (m, 1H), 1.31 (m, 2H), 1.15 (m, 2H).

### Example 10

### 7-[4-(5-Chloro-1-oxo-indan-2-ylmethyl)-piperazin-1-yl]-1-cyclopropyl-6-fluoro-4-oxo-1,4-dihydro-quinoline-3-carboxylic acid (Compound 10)

¹H NMR (300 MHz, CDCl₃): δ 15.06 (s, 1H), 8.78 (s, 1H), 8.02 (d, *J* = 12 Hz, 1H), 7.78 (s, 1H), 7.3 (d, *J* = 9.0 Hz, 1H), 6.83 (dd, *J* = 8.7 and 2.3 Hz, 1H ), 6.70 (d, *J* = 2.22 Hz, 1H), 3.51 (m, 1H), 3.34 (br s, 4H), 3.01 (m, 1H), 2.98 (m, 1H), 2.75 (m, 2H), 2.62 (br s, 4H), 1.60(m, 1H), 1.32 (m, 2H), 1.18 (m, 2H).

### Example 11

### 7-[4-(8-Chloro-5-oxo-2,3,4,5-tetrahydro-benzo[b]thiepin-4-ylmethyl)-piperazin-1-yl]-1-cyclopropyl-6-fluoro-4-oxo-1,4-dihydro-quinoline-3-carboxylic acid (Compound 11)

¹H NMR (300 MHz, DMSO-*d*₆): δ 15.1 (br s, 1H), 8.42 (s, 1H), 7.72 (d, *J* = 13.6 Hz, 1H), 7.58 (m, 2H), 7.41-7.35 (m, 2H), 4.72 (m, 1H), 4.60 (m, 1H), 3.28 (br s, 4H), 3.04 (m, 3H), 2.87 (m, 1H), 2.65 (m, 1H), 2.49 (br s, 4H), 1.61 (m, 1H), 1.35 (m, 2H), 1.20 (m, 2H).

### Example 12

### 7-[4-(8 -Chloro-5-oxo-2,3,4,5-tetrahydro-benzo[b]thiepin-4-ylmethyl)-piperazin-1-yl]-1-ethyl-6-fluoro-4-oxo-1,4-dihydro-quinoline-3-carboxylic acid (Compound 12)

¹H NMR (300 MHz, DMSO-*d*₆): δ 15.1 (br s, 1H), 8.42 (s, 1H), 7.72 (d, *J* = 13.6 Hz, 1H), 7.58 (m, 2H), 7.41-7.35 (m, 2H ), 4.74 (m, 2H), 4.72 (m, 1H), 4.60 (m, 1H), 3.28 (br s, 4H), 3.04 (m, 3H), 2.87 (m, 1H), 2.65 (m, 1H), 2.49 (br s, 4H), 1.39 (t, 3H).

### Example 13

### 1-Cyclopropyl-6-fluoro-7-[4-(1-hydroxyimino-6-methoxy-1,2,3,4-tetrahydro-naphthalen-2-ylmethyl)-piperazin-1-yl]-4-oxo-1,4-dihydro-quinoline-3-carboxylic acid (Compound 13)

To a solution of 1-cyclopropyl-6-fluoro-7-[4-(6-methoxy-1-oxo-1,2,3,4-tetrahydro-naphthalen-2-yl methyl)-piperazin-1-yl]-4-oxo-1,4-dihydro-quinoline-3-carboxylic acid (Compound 1) (0.1 g, 0.192 mmol) in ethanol (5 mL) was added hydroxylamine hydrochloride (0.66 g, 0.962 mmol) and pyridine (0.5 mL) at 26-27 °C. Reaction mixture was refluxed for 2 h at 76-77 °C. Progress of reaction was checked by TLC using 10 % methanol in chloroform as mobile phase. The solvents were concentrated on a rotatory evaporator under reduced pressure. The residue was diluted with water (10 mL) and extracted with dichloromethane (2 x 5 mL). The organic layer was separated, dried over anhydrous Na₂SO₄ and concentrated on a rotatory evaporator to afford 1-cyclopropyl-6-fluoro-7-[4-(1-hydroxyimino-6-methoxy- 1,2,3,4-tetrahydro-naphthalen-2-ylmethyl)-piperazin-1-yl]-4-oxo-1,4-dihydro-quinoline-3-carboxylic acid as a white solid (0.05 g, 45 %).
¹H NMR (300 MHz, CDCl₃): δ 15.05 (br s, 1H), 9.08 (s, 1H), 8.71 (s, 1H), 7.91 (d, *J* = 4.29 Hz, 1H), 7.82 (s, 1H), 7.33 (d, *J* = 7.14 Hz, 1H), 6.80 (dd, *J* = 8.73 and 2.43 Hz, 1H ), 6.62 (d, *J* = 2.22 Hz, 1H), 3.80 (s, 3H), 3.46 (m, 1H), 3.27 (br s, 4H), 2.91 (m, 1H), 2.87 (m, 2H), 2.64 (m, 2H), 2.51 (br s, 4H), 2.33 (m, 1H), 1.56 (m, 1H), 1.29 (m, 2H), 1.16 (m, 2H).

### Biological activity:

### Determination of In vitro MIC

The *in vitro* (MIC) antibacterial activity of the compounds against susceptible Gram-positive, multidrug resistant Gram-positive and Gram-negative was tested as growth inhibition with the use of broth micro dilution method according to the guidelines of Clinical and Laboratory Standards Institute (formerly National Committee for Clinical Laboratory Standards). Compounds were dissolved in DMSO and water was added to get stock solution in 80% DMSO. The working solution was prepared by diluting the stock solution 1:10 times in 4-8 % DMSO in water or medium, and the dissolved compounds were evaluated in the concentrations of 0.125, 0.25, 0.5, 1, 2, 4, 8, and 16 µg/mL and the concentration was finally expressed in µM.

**Table 1 In Vitro MIC values (µM) of novel quinolones against various susceptible, resistant Gram-positive bacteria and Gram-negative bacteria**

| Sr. | Bacterial Strain | Compound **1** | Compound **6** | Ciprofloxacin | Norfloxacin |
|---|---|---|---|---|---|
| 1 | *Bacillus cereus* **ATCC 11778** | 3.125 | 1.563 | 1.563 | 3.125 |
| 2 | *Staphylococcus aureus* **ATCC 25923** | 1.563 | 0.781 | 0.781 | 3.125 |
| 3 | *Staphylococcus aureus* **ATCC 29213** | 3.125 | 1.563 | 0.781 | 3.125 |
| 4 | *Enterococcus faecalis* **ATCC 14506** | 12.5 | 6.25 | 6.25 | 25 |
| 5 | *Klebsiella pneumoniae* **ATCC 10031** | ≤0.391 | ≤0.391 | ≤0.391 | ≤0.391 |
| 6 | *Pseudomonas aeruginosa* **ATCC 27853** | 3.125 | 0.781 | 0.781 | 3.125 |
| 7 | *Staphylococcus epidermidis* **ATCC 155** | 0.781 | ≤0.391 | ≤0.391 | 0.781 |
| 8 | *Streptococcus pyogens* **ATCC 14289** | 1.563 | 0.781 | 0.781 | 3.125 |
| 9 | *Enterococcus faecalis* **ATCC 35550** | 3.125 | 6.25 | 1.563 | 6.25 |
| 10 | *Staphylococcus aureus* **ATCC 9144** | 1.563 | 0.781 | 0.781 | 1.563 |
| 11 | *Staphylococcus aureus* **ATCC 14154** | 1.563 | 1.563 | 0.781 | 3.125 |
| 12 | *Bacillus pumilis* **ATCC 14884** | 1.563 | 0.781 | 0.781 | 6.25 |
| 13 | *Staphylococcus aureus* **ATCC 700699** | >50 | 25 | 50 | >50 |
| 14 | *Enterococcus faecalis* **ATCC 700802** | 12.5 | 6.25 | 6.25 | 25 |
| 15 | *Streptococcus pneumoniae* **ATCC 700904** | 3.125 | 3.125 | 0.781 | 1.563 |
| 16 | *Enterococcus faecium* **ATCC 700221** | 25 | 50 | >50 | >50 |
| 17 | *Enterococcus faecium* **ATCC 51559** | >50 | 50 | 50 | >50 |
| 18 | *Staphylococcus pyogens* **ATCC 700603** | 12.5 | 12.5 | 25 | >50 |
| 19 | *Klebsiella pneumoniae* **ATCC 700571** | 6.25 | 1.563 | 0.781 | 6.25 |

### Pharmacokinetics:

Pharmacokinetics of the test compounds **1**, **6** and ciprofloxacin was studied (Table 2) *via* per-oral and intravenous routes of administration in male Sprague-Dawley rats of 8 to 10 weeks of age. Animals were fasted for 18 h and food was supplied after 4.0 h of administration of the test compound. There was free access to water throughout the study. A homogenous suspension of the test substance was prepared in 0.5 % w/v CMC in normal saline and a per-oral dose of 30 mg/kg was administered. After the administration of the test compounds, blood samples were withdrawn at various time intervals through retro-orbital plexus and collected into heparinized micro centrifuge tubes. Plasma was separated by centrifugation at 4000 rpm for 5 min at ambient temperature and analyzed immediately. Remaining samples were stored at -20 °C until analyzed. Analysis was carried out by taking an aliquots of 180 µL plasma and 20 µL of internal standard (Atorvastatin) and was extracted with 2.5 mL of extracting solvent (ethyl acetate: acetonitrile, 80:20, v/v) in glass test-tube by vortexing with spinix vortex mixture for a minute. This was then centrifuged at 2000 rpm for 2.0 min. The supernatant was transferred to another glass test-tube and the solvent was evaporated under nitrogen using Zymark evaporator at 40 °C. Finally, the tubes were reconstituted with 0.1 mL diluent (acetonitrile: methanol: water: 40:40:20). The reconstituted samples were analyzed on Agilent 1100 Series HPLC system with a mobile phase of 0.05 % v/v trifluoroacetic acid in water: acetonitrile (32:68, v/v); flowing at a flow rate of 1.0 mL/min through a Kromasil 250 mm x 4.6 mm x 5 µm column maintained at 30 °C. Chromatographic separation was achieved within 15 min. Agilent software version Chemstation Rev.A.09.01. (1206) was used to acquire and process all chromatographic data. Quantification was based on a series of calibrators ranging from 0.031 to 32 µg/mL, prepared by adding test compound to drug free rat plasma. Quality control samples were analyzed in parallel to verify that the system performs in control. Pharmacokinetic parameters namely; maximum plasma concentration (Cₘₐₓ), time point of maximum plasma concentration (tₘₐₓ), area under the plasma concentration-time curve from 0 h to infinity (AUC_{0-∝}) and half-life of drug elimination during the terminal phase (t_{1/2}) were calculated from plasma concentration *versus* time data, by standard non-compartmental methods, using the WinNonLin software version 4.0.1 procured from Pharsight

**Table 2. Mean pharmacokinetic parameters of 1, 6 and Ciprofloxacin in fasted male SD rat.^{a}**

| Compd. | Route | dose | Tₘₐₓ | Cₘₐₓ | T_{1/2} | AUC(0-∝**)** |
|---|---|---|---|---|---|---|
| | | (mg/kg) | (h) | (µg/MI) | (h) | (h.µg/mL) |
| **1** | Oral | 30 | 4.00 ± 0.00 | 10.56 ± 2.61 | 9.55 ± 2.83 | 85.76 ± 39.94 |
| **6** | Oral | 30 | 1.33 ± 0.53 | 2.36 ± 1.07 | 1.66 ± 0.73 | 7.40 ± 1.78 |
| **Ciprofloxacin** | Oral | 30 | 0.44 ± 0.20 | 1.49 ± 0.22 | 1.90 ± 0.30 | 4.65 ± 0.16 |

| | | | | | | |
|---|---|---|---|---|---|---|
| ^{a}Values indicate mean ± SD for n-3 rats | | | | | | |

The compounds of the invention were found to be effective and had very little adverse events. These compounds therefore have the potential to be antibacterial compounds having superior beneficial effects over other known antibacterial compounds of this class.

## Claims

1. Novel quinolone compounds represented by general formula (I), and pharmaceutically acceptable salts thereof, 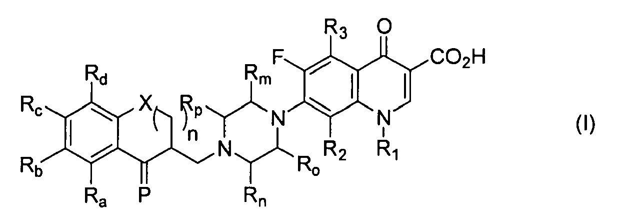 wherein R₁ represents hydrogen, linear or branched, substituted or unsubstituted groups selected from (C₁-C₁₂) alkyl, (C₃-C₁₂)cycloalkyl groups; R₂ and R₃ may be same or different and independently represent H, OH, halogen, alkoxy or groups selected from NO₂, CN; Rₘ, Rₙ, Rₒ and Rₚ may be same or different and independently represents hydrogen or alkyl groups; Rₐ, R_{b}, R_{c}, R_{d} may be same or different and independently represents hydrogen, halogen, haloalkyl, hydroxy, alkoxy, thio, nitro, cyano, or substituted or unsubstituted groups selected from amino, linear or branched (C₁-C₁₂)alkyl, (C₁-C₁₂)alkoxy derivatives of sulfenyl and sulfonyl groups, sulfonic acid and its derivatives; 'P' represents oxygen, sulfur or the group representing -NR, where R represents a -H, -OH or a (C₁-C₃) alkyl group which may be optionally substituted; 'X' may be absent or present and if present, represents the groups -CH₂-, -O-, -S, SO or SO₂; n represents an integer from 0-3.

2. A compound as claimed in claim 1 wherein said suitable substituents comprise one or more unsubstituted or substituted radicals selected from the group consisting of hydroxyl, halo, thio, nitro, amino, cyano, alkyl, haloalkyl, alkoxy, haloalkoxy, cycloalkyl, alkoxy, aryl, aryloxy, sulfenyl derivatives, sulfonyl derivatives, sulfonic acid and its derivatives, phosphonic acid and its derivatives.

3. A compound as claimed in claim 1 or 2 wherein when any of the substituents are further substituted, the further substituents comprising one or more radicals selected from the group consisiting of hydroxyl, halo, thio, nitro, amino, cyano, alkyl, haloalkyl, alkoxy, haloalkoxy, alkenyl, alkoxy, aryl, acyl, sulfenyl derivatives, sulfonyl derivatives, sulfonic acid and its derivatives, phosphonic acid and its derivatives.

4. A compound according to any one of the preceding claims, wherein R₁ is an unsubstituted C₁-C₆ alkyl or C₃-C₆ cycloalkyl group, preferably an unsubstituted C₁-C₄ alkyl or cyclopropyl group.

5. A compound according to claim 4, wherein R₁ is an unsubstituted ethyl or cyclopropyl group.

6. A compound according to any one of the preceding claims, wherein R₂ is an unsubstituted hydrogen, halogen or C₁-C₄ alkoxy group, preferably a hydrogen, halogen or C₁-C₂ alkoxy group, and/or R₃ is hydrogen or halogen.

7. A compound according to any one of the preceding claims, wherein Rₘ, Rₙ, Rₒ and Rₚ are the same or different and each represent hydrogen or a C₁-C₄ alkyl group, and preferably Rₘ, Rₙ and Rₒ are all hydrogen and Rₚ is hydrogen or C₁-C₄ alkyl.

8. A compound according to any one of the preceding claims, wherein X is CH₂, S or O and/or P is O, S or N-OH and/or n is 0, 1 or 2.

9. A compound according to any one of the preceding claims, wherein Rₐ to R_{d} are the same or different and each represent hydrogen, halogen or C₁-C₄ alkoxy, and preferably Rₐ, R_{b} and R_{d} are all hydrogen and R_{c} represents halogen or C₁-C₄ alkoxy.

10. A compound as claimed in any preceding claim, selected from
1-Cyclopropyl-6-fluoro-7-[4-(6-methoxy-1-oxo-1,2,3,4-tetrahydro-naphthalen-2-yl methyl)-piperazin-1-yl]-4-oxo-1,4-dihydro-quinoline-3-carboxylic acid
1-Cyclopropyl-6-fluoro-8-methoxy-7-[4-(6-methoxy-1-oxo-1,2,3,4-tetrahydro-naphthalen-2-ylmethyl)-piperazin-1-yl]-4-oxo-1,4-dihydro-quinoline-3-carboxylic acid
1 -Cyclopropyl-5,6,8-trifluoro-7-[4-(6-methoxy-1-oxo-1,2,3,4-tetrahydro-naphthalen-2-ylmethyl)-piperazin-1-yl]-4-oxo-1,4-dihydro-quinoline-3-carboxylic acid
1-Cyclopropyl-6-fluoro-8-methoxy-7-[4-(6-methoxy-1-oxo-1,2,3,4-tetrahydro-naphthalen-2-ylmethyl)-3-methyl-piperazin-1-yl]-4-oxo-1,4-dihydro-quinoline-3-carboxylic acid
1-Ethyl-6-fluoro-7-[4-(6-methoxy-1-oxo-1,2,3,4-tetrahydro-naphthalen-2-ylmethyl)-piperazin-1-yl]-4-oxo-1,4-dihydro-quinoline-3-carboxylic acid
7-[4-(8-Chloro-5-oxo-2,3,4,5-tetrahydro-benzo[b]oxepin-4-ylmethyl)-piperazin-1-yl]-1 -cyclopropyl-6- fluoro-4-oxo-1,4-dihydro-quinoline-3-carboxylic acid
7-[4-(8-Chloro-5-oxo-2,3,4,5-tetrahydro-benzo[b]oxepin-4-ylmethyl)-piperazin-1-yl]-1-ethyl-6-fluoro-4-oxo-1,4-dihydro-quinoline-3-carboxylic acid
7-[4-(7-Chloro-4-oxo-chroman-3-ylmethyl)-piperazin-1-yl]-1-cyclopropyl-6-fluoro-4-oxo-1,4-dihydro-quinoline-3-carboxylic acid
7-[4-(7-Chloro-4-oxo-chroman-3-ylmethyl)-piperazin-1-yl]-1-ethyl-6-fluoro-4-oxo-1,4-dihydro-quinoline-3-carboxylic acid
7-[4-(5-Chloro-1-oxo-indan-2-ylmethyl)-piperazin-1-yl]-1-cyclopropyl-6-fluoro-4-oxo-1,4-dihydro-quinoline-3-carboxylic acid
7-[4-(8-Chloro-5-oxo-2,3,4,5-tetrahydro-benzo[b]thiepin-4-ylmethyl)-piperazin-1-yl]-1-cyclopropyl-6-fluoro-4-oxo-1,4-dihydro-quinoline-3-carboxylic acid
7-[4-(8-Chloro-5-oxo-2,3,4,5-tetrahydro-benzo[b]thiepin-4-ylmethyl)-piperazin-1-yl]-1-ethyl-6-fluoro-4-oxo-1,4-dihydro-quinoline-3-carboxylic acid
1-Cyclopropyl-6-fluoro-7-[4-(1-hydroxyimino-6-methoxy-1,2,3,4-tetrahydro-naphthalen-2-ylmethyl)-piperazin-1-yl]-4-oxo-1,4-dihydro-quinoline-3-carboxylic acid
and pharmaceutically acceptable salt thereof.

11. A pharmaceutical composition which comprises a compound of formula (1), as defined in any preceding claim, or a pharmaceutically acceptable salt thereof, and a pharmaceutically acceptable carrier, diluent or excipient.

12. A compound of formula (1), as defined in any one of claims 1 to 10, or a pharmaceutically acceptable salt thereof, or a pharmaceutical composition or medicine containing the compound or salt, for use as an antibiotic.

13. A process for preparing compounds of general formula (I) comprising,
a) stirring compound of general formula (III) with aqueous formaldehyde solution in suitable solvent(s) at suitable temperature and concentrating the reaction mixture
b) treating the residue of above step with HCl, removing the solvent under reduced pressure and treating the residue with a suitable ketone of general formula (II) in suitable solvent(s), and the resulting mixture is refluxed, the said suitable solvent(s) being selected from methanol, ethanol, propanol, isopropanol, butanol, isobutanol, t-butanol or mixtures thereof.

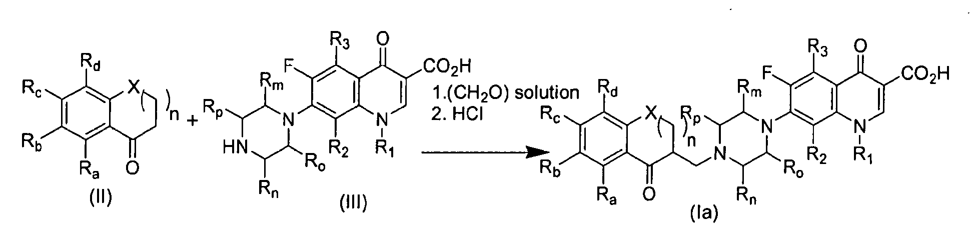

14. A process for preparing compounds of general formula (I) comprising
a) stirring compound of general formula (Ia) with suitable amine in suitable solvent(s) selected from methanol, ethanol, propanol, isopropanol, butanol, isobutanol, t-butanol or mixtures thereof at suitable temperature,
b) concentrating the resulting mixture.

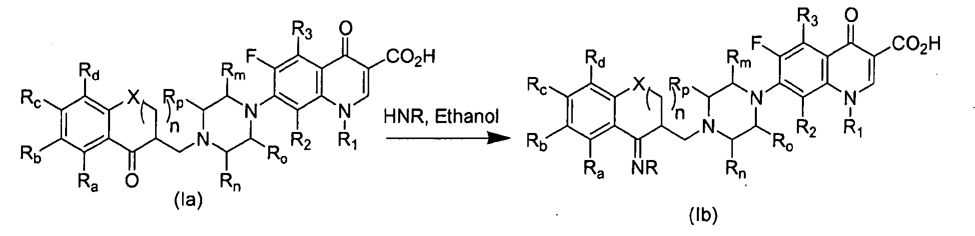

15. A process for preparing compounds of general formula (I) comprising
a) refluxing compound of general formula (Ia) with Lawesson's reagent in suitable solvent(s) selected from THF, toluene, propanol, isopropanol or mixtures thereof at suitable temperature.
b) concentrating the resulting mixture followed by purification
